(19) **Europäisches Patentamt / European Patent Office / Office européen des brevets**

(11) **EP 2 163 539 B1**

(12) **EUROPEAN PATENT SPECIFICATION**

(45) Date of publication and mention
of the grant of the patent:
**22.06.2016 Bulletin 2016/25**

(21) Application number: **08752453.4**

(22) Date of filing: **08.05.2008**

(51) Int Cl.:
*C07C 227/42* (2006.01)      *C07B 63/00* (2006.01)
*C07C 229/24* (2006.01)

(86) International application number:
**PCT/JP2008/058567**

(87) International publication number:
**WO 2008/140023 (20.11.2008 Gazette 2008/47)**

(54) **METHOD FOR PRECIPITATING alpha-CRYSTALS OF GLUTAMIC ACID**

VERFAHREN ZUR PRÄZIPITATION VON alpha-KRISTALLEN VON GLUTAMINSÄURE

PROCÉDÉ DE PRÉCIPITATION DE CRISTAUX alpha D'ACIDE GLUTAMIQUE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **14.05.2007 JP 2007127524**

(43) Date of publication of application:
**17.03.2010 Bulletin 2010/11**

(73) Proprietor: **Ajinomoto Co., Inc.**
**Chuo-ku, Tokyo 104-8315 (JP)**

(72) Inventors:
• **NAGAI, Hidetada**
**Kawasaki-shi**
**Kanagawa 210-8681 (JP)**

• **TAKAHASHI, Yoshifumi**
**Kawasaki-shi**
**Kanagawa 210-8681 (JP)**
• **YAMANE, Takao**
**Kawasaki-shi**
**Kanagawa 210-8681 (JP)**
• **KONISHI, Nobuharu**
**Kawasaki-shi**
**Kanagawa 210-8681 (JP)**

(74) Representative: **Strehl Schübel-Hopf & Partner**
**Maximilianstrasse 54**
**80538 München (DE)**

(56) References cited:
**CN-A- 1 312 245     JP-B1- 45 004 730**
**US-A- 5 907 059**

## Description

### Technical Field

This invention relates to a crystallization method which precedes to crystallize $\alpha$-form crystals which are metastable crystals in the polymorphism of glutamic acid crystals existing two forms.

### Background Art

In glutamic acid crystals, polymorphism of two forms exist, one is $\alpha$-form which is metastable form, and the other is $\beta$-form which is stable form.

The presence of $\alpha$-form crystals has been known for a long time (Patent Document 1). It is also known to purify glutamic acid by depositing once $\alpha$-form crystals, and then transferring them to $\beta$-form crystals (Patent Document 2). This is considered due that impurities included in $\alpha$-form crystals are released during transformation of crystals.

As the production of $\alpha$-form crystals, it can be considered that supersaturated state is made by adding an acid to a glutamic acid salt solution to decrease solubility of glutamic acid, and $\alpha$-form crystals are added thereto as seed crystals, similar to a method of adding $\alpha$-form crystals in glycine (Non-Patent Document 1). Another method is also known of producing $\alpha$-form crystals by adding amino acids or sugars (Patent Documents 1, 3). In addition, it is scientifically known to deposit first $\alpha$-form crystals while forming supersaturation and being allowed to stand without adding seed crystals (Non-Patent Document 2). However, a crystallization method of producing only $\alpha$-form crystals by utilizing this phenomenon has not been known yet.

[Patent Document 1] JP36-017712B
[Patent Document 2] JP45-004730B
[Patent Document 3] JP45-011286B
[Non-Patent Document 1] N. Doki et al., Crystal Growth & Design, 4(5), 949-953, 2004
[Non-Patent Document 2] Jochen Scholl et al., Chem. Eng. Technol. 29(2), 257-264,2006

Incidentally, the method of producing $\alpha$-form crystals by adding seed crystals has the following problems. That is , (1) it is difficult to store prepared seed crystals so as not to transfer them to $\beta$-form crystals by rendering moisture adhered to separated crystals little. Furthermore, (2) it is also difficult to control a glutamic acid salt solution to a prescribed rate of supersaturation, because in the range of pH 4-6, solubility of glutamic acid is highly sensitive with pH. That is, upon pH adjustment, when pH becomes lower than a prescribed value caused by too fast addition of acid, $\beta$-form crystals which are stable form crystals deposit due to a high rate of supersaturation. Whereas, when pH becomes too high, added seed crystals are dissolved, and accordingly, effects of seed crystals cannot be expected at all resulting in deposition of $\beta$-form crystals.

The method of producing $\alpha$-form crystals by adding amino acids or sugars results in addition of impurities to the system, and accordingly, it does not meet the object of purification wherein removal of impurities is a primary function of crystallization.

CN 1312245A discloses a process in which a solution of glutamic acid at pH 6.7 is added to a solution of glutamic acid at pH 1.5 until a mixture at the isoelectric point of glutamic acid is obtained.

GB 1118942 discloses a process for converting the $\alpha$-form of glutamic acid crystals into the $\beta$-form.

An object of the invention is to provide a neutralization crystallization method for initially depositing $\alpha$-form crystals which are metastable crystals with good reproducibility without depositing $\beta$-form crystals which are in stable form, without adding $\alpha$-form crystals or additives for preceding crystallization of $\alpha$-form crystals.

### Disclosure of The Invention

In neutralization crystallization for depositing $\alpha$-form crystals, several cases can be considered as to deposition mechanism of $\beta$-form crystals. The first case is that $\beta$-forum crystals deposit independently as primary nuclei generation from the solution phase, even when $\alpha$-form crystals are depositing. The second case is that once $\alpha$-form crystals deposit, and then, they are transformed into $\beta$-form crystals. The third case is that deposited $\alpha$-form crystals induce secondary nuclei generation of $\beta$-form crystals as seed crystals. The inventors investigated the deposition mechanism widely, and in solutions with low rate of supersaturations (1.0-1.7), deposition of crystals was observed. As a result, deposition of $\alpha$-form crystals was found and simultaneously, $\beta$-form crystals were also observed not in the vicinity of $\alpha$-form crystals but in the bulk solution phase. Therefore, they considered that $\beta$-form crystals deposit independently from the solution phase by primary nuclei generation. They conceived that it is possible to prevent deposition of $\beta$-form crystals by depositing α-form crystals to allow supersaturation disappear prior to the deposition of β-form crystals.

The present invention provides the following:

[1] A method of producing α-form glutamic acid crystals which comprises the steps of:

(A) forming a first supersaturated state of a glutamic acid salt solution having a rate of supersaturation of 1.0 to 1.7 by adding an acid at once to lower pH of the solution beyond isoelectric point of glutamic acid to pH 1.3 to 2.0 and waiting at least 1 minute after the addition of acid to the glutamic acid salt solution; and

(B) forming a second supersaturated state of the solution by adding a glutamic acid salt solution within 25 minutes after the first supersaturated state is formed to shift the pH toward the isoelectric point.

[2] The method of [1] above, which is performed without adding seed crystals.

[3] The method of [1] above, which comprises

preparing a glutamic acid salt solution and an acid with a volume capable of neutralizing the glutamic acid dissolved in the solution to its isoelectric point of pH 3.2,

dividing the glutamic acid salt solution into two portions, using one of the portions as the glutamic acid salt solution in the step (A) to form the first supersaturated state by adding the above acid, and

using the other portion as the glutamic acid salt solution in the step (B).

[4] A method of producing monosodium glutamate monohydrate which comprises transforming α-form glutamic acid crystals produced in the method of [1] above to β-form glutamic acid crystals, dissolving the β-form glutamic acid crystals by sodium hydroxide aqueous solution to adjust to a pH forming monosodium glutamate monohydrate, concentrating to deposit crystals, and drying the deposited crystals.

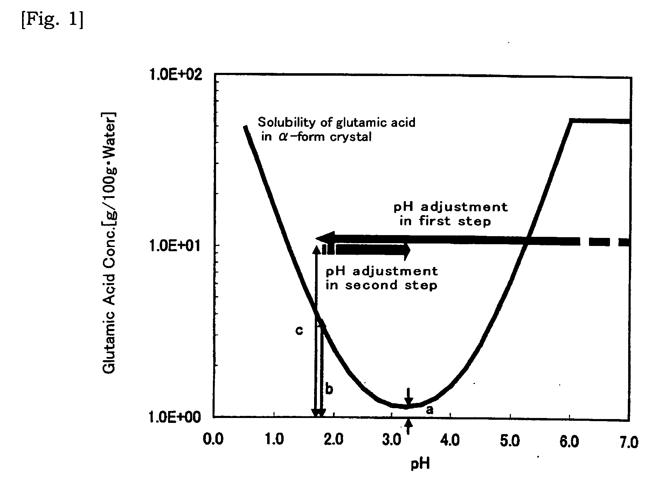

In the crystallization method of the invention, an aqueous solution containing glutamic acid is prepared, and a volume of acid capable of neutralizing whole quantity of glutamic acid contained therein is metered. If the total volume of the acid prepared is added to a part of glutamic acid, pH becomes lower than the isoelectric point of glutamic acid of pH 3.2. In this region, as shown in Figure 1, the lower the pH is, the greater the solubility. Accordingly, when the initial glutamic acid concentration exceeds the solubility, supersaturation can be made. When the supersaturation in the first step is kept at a given level, after a time elapsed, β-form crystals or a mixture of α-form and β-form crystals deposit. Then, there is a need to deposit α-form crystals preferentially by applying supersaturation in the second step prior to the deposition of β-form crystals. For this purpose, the remainder of the glutamic acid aqueous solution, of which a part has been used, is added to the supersaturated aqueous solution produced in the first step to form the second step supersaturation. Since to the solution itself, an amount of acid necessary for the adjustment to isoelectric point of glutamic acid has to be added, the pH of the solution becomes 3.2 at this time to reach a maximum crystallization rate.

According to the invention, α-form crystals of glutamic acid can be produced easily and stably in a short period.

Brief Description of The Drawing

[Figure 1] It is a diagrammatic illustration illustrating the two-step neutralization crystallization method of the invention.

[Figure 2] It is a graph showing a relationship between the rate of supersaturation in the first step neutralization, the time up to the second step neutralization, and the form of deposited crystals, in the case that overall rate of supersaturation is 8.0, obtained in the method of the invention.

[Figure 3] It is a graph showing a relationship between the rate of supersaturation in the first step neutralization, the time up to the second step neutralization, and the form of deposited crystals, in the case that overall rate of supersaturation is 4.0, obtained in the method of the invention.

[Figure 4] It is a graph which indicates a result of determination of appearing time of β-forum crystal based on temperature and rate of supersaturation from the results of Figures 2 and 3.

[Figure 5] It is microphotographs of glutamic acid crystals obtained in Examples.

[Figure 6] It shows spectra of glutamic acid crystals obtained in Examples measured by Raman spectrophotometry.

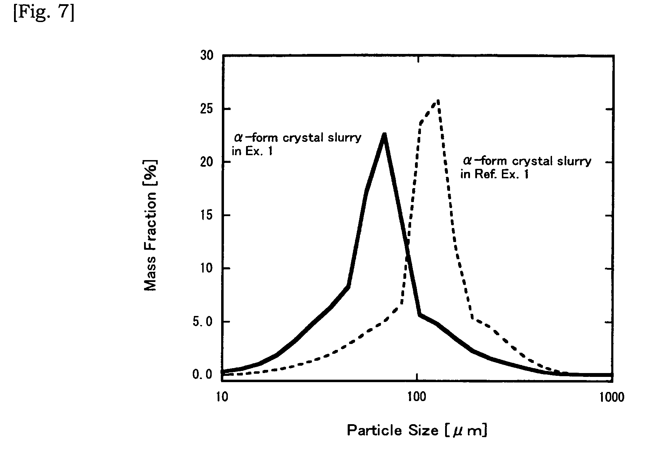

[Figure 7] It is a graph indicating particle size distribution of crystals obtained in Example 1 of the invention and Reference Example 1.

Best Mode for Carrying Out The Invention

The initial glutamic acid concentration in this crystallization is desirably 50 g/l to 200 g/l, preferably 80 to 120 g/l. Type of cation forming salt of glutamic acid in the glutamic acid salt solution is not limited, and commonly, is ammonium ion or sodium ion. PH of the solution is, in general, in the range of about 5.5 to 8.0, frequently about 6.0 to 7.5. Illustrative of the glutamic acid salt solutions are glutamic acid fermentation broths, solutions from which bacterial cells have been removed, and the like.

Every acid functionable as hydrogen ion donor, such as hydrochloric acid, sulfuric acid and phosphoric acid, is usable as the acid to be added to the glutamic acid salt solution. Preferred volume of the acid is able to neutralize the glutamic acid dissolved in the glutamic acid salt solution up to its isoelectric point of pH 3.2, in order to raise a final crystallization rate of glutamic acid, and in practical viewpoint, in the range of the volume to neutralize up to pH 3.2 $\pm$ 3%, preferably $\pm$ 1%.

The above glutamic acid salt solution is divided into two portions, and one of them is used in the step of forming the first supersaturated state. The volume of the glutamic acid salt solution used in the first step is set so that the rate of supersaturation in the first supersaturated state is 1.0 to 1.7. Out of the above range, it is possible to deposit β-form crystals within 25 minutes.

In general crystallization operation, a solution of a solute to be deposited is prepared in a high solubility range of the solute, and then, the solution is placed lower solubility conditions than the initial concentration by controlling temperature, pH or the like of the solution to form supersaturation, and it is utilized as a driving force for the deposition of crystals. The supersaturation means a temporary dissolved state under a crystallization operation, in spite that the concentration of a solute is higher than its solubility. The magnitude of supersaturation is expressed in two ways, one is called rate of supersaturation, and the other is called degree of supersaturation. The rate of supersaturation is given by a division actual concentration of a solute in its solution state divided by its solubility. The degree of supersaturation is the remainder obtained by subtracting the solubility from actual concentration of a solute. In this specification the magnitude of supersaturation is expressed by the rate of supersaturation.

In order to calculate the rate of supersaturation applied in the first supersaturated state, the solubility of glutamic acid is calculated by using the following approximations. The temperature dependency of the solubility of glutamic acid has already been known (Non-Patent Document 3), and it is approximated by the Clausius-Clapeyron's formula to obtain;

[Mathematical 1]

$$\text{Solubility at } pH\,3.2(s)[g\,/100g\ \text{water}\,] = \exp\left[\frac{-3348.1}{T[\text{°C}]+273} + 11.375\right]$$

The pH dependency of the solubility of glutamic acid has also been known (Non-Patent Document 3), and it is approximated by the following formula;

[Mathematical 2]

$$\text{Solubility } (s, pH)[g\,/100g\ \text{water}\,]$$
$$= \text{Solubility at pH3.2(S)[g/100g water]} \times \frac{84.6}{\text{existing ration of Glu}^{\pm}\text{at each pH}}$$

Hereupon, Glu $\pm$ represents glutamate ion having electric charge of zero. The hydrogen ion dissociation constants are known to be $pk_1$=2.19, $pk_2$=4.25, $pk_3$=9.67, respectively (Non-Patent Document 4) Conversion of unit from solubility per 100g water to g/l is carried out by the following formula;

[Mathematical 3]

$$\text{Solubility (s, pH)[g/100g water]} = \frac{\text{Solubility (s, pH)[g/100g water]}}{\text{Solubility (s, pH)[g/100g water]}+100} \times 1000 \times 1.06$$

provided that solid matter is glutamic acid alone, and that specific gravity is d= 1.06g/ml. Using the solubility values determined as above, the rate of supersaturation is calculated. In Figure 1, the rate of supersaturation in the first supersaturated state is represented by c/b, and that in the second supersaturated, i.e. overall rate of supersaturation is represented by c/a.

[Non-Patent document 3] "Shokuhin Kogyo Handbook", p.651-656, Kenpokusha, 2004
[Non-Patent document 4] "Kagaku Binran", p.432, Maruzen, 1993

The volume of aqueous glutamic acid salt solution used for forming the first supersaturated state is determined by adding the glutamic acid salt solution with varying volume to prepare a calibration curve of pH previously , and then determining the volume necessary for rendering the solution to a desired pH. That is, a pH which makes a desired rate of supersaturation is determined, and the volume necessary therefor is found from the calibration curve. Therefore, a range of the pH results in depending on the glutamic acid concentration of the aqueous solution prior to the deposition of crystals. The pH range corresponding to the range of the rate of supersaturation in the first supersaturated state of 1.0 to 1.7 is 1.3 to 1.6 in the crystallization at an overall rate of supersaturation of 8.0.

The first supersaturated state can be formed by adding the aforementioned acid to the above volume of the glutamic acid salt solution. A desirable liquid temperature is in the range of 20 to 30°C. Risk of depositing β-form crystals increases whichever the temperature is located higher than or lower than this range. The acid is added at once. While, the solution is necessary to be stirred to homogenize it.

In the invention, in order to deposit α-form crystals preferentially, the remaining glutamic acid salt solution must be added within a definite time after the first supersaturated state is formed. The definite time was determined, for example as follows:

100ml of 127g/l monosodium glutamate monohydrate (MSG) aqueous solution was prepared, and 3.4g of sulfuric acid was taken up in a beaker. The quantity of glutamic acid was 0.068 mole and sulfuric acid was 0.034 mole. With varying the neutralizing volume in the first step, the MSG aqueous solution was kept at 25°C in a water bath while stirring by a magnetic stirrer, and the sulfuric acid was mixed in a moment. With varying time, the remaining MSG aqueous solution was added after the time to apply the supersaturation in the second step. At that time, overall rate of supersaturation was 8.0.

The judgment of the form of deposited glutamic acid crystals, whether α-form crystal or β-form crystal, was conducted by observation through a microscope. When the judgment by microscope was difficult, the crystal form was judged, whether it is β-form or not, by conducting recrystallization using the slurry as seed crystals. In concrete, a 127g/l MSG solution was prepared, and 1ml of the solution was taken up. To this solution, about $200\mu l$ sample for judgment was added. Thereafter, $20\mu l$ concentrated sulfuric acid was added immediately, and shaken by hand. The resulting crystals were observed by a microscope to determine whether β-form crystals were contaminated or not.

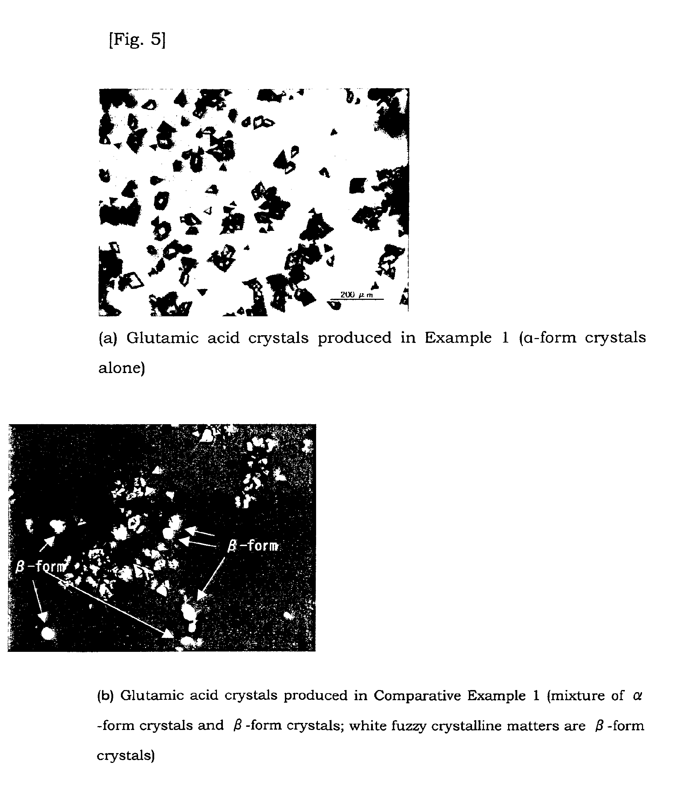

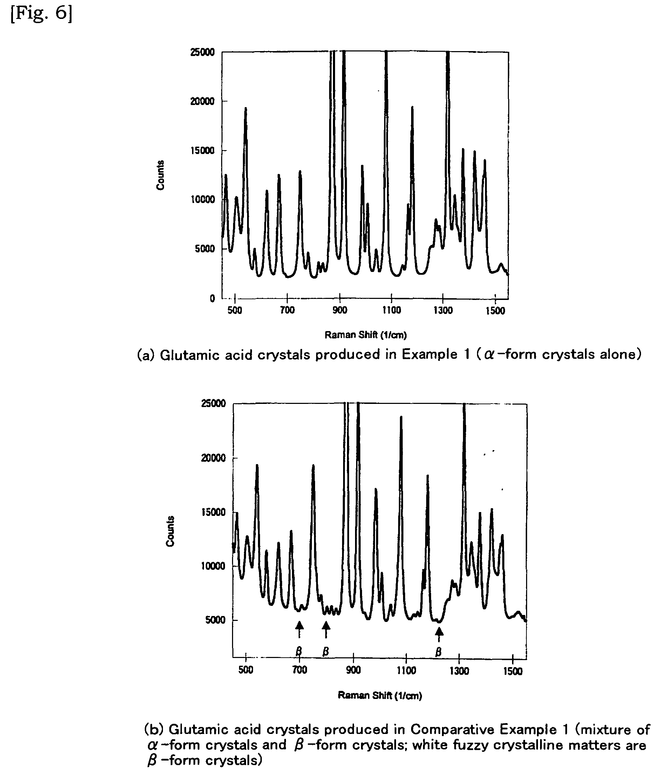

The metastable crystal (α-form crystal) of glutamic acid has a shape as in the microphotograph of Figure 5 (a), and shows a chart as in Figure 6 (a) by the analysis by a Raman spectrometer. While, the stable crystal is a flat crystal as in Figure 5(b), and shows characteristic peaks as in Figure 6(b).

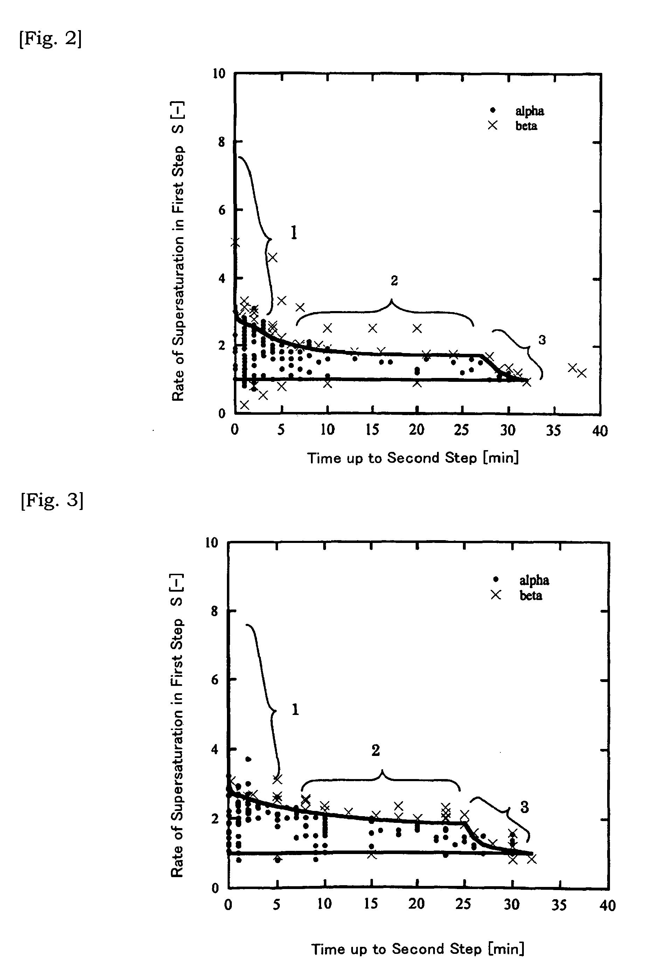

The relationships between the rate of supersaturation in the first supersaturation state, the time from the addition of sulfuric acid to the addition of remaining MSG aqueous solution, and the form of deposited crystals which are in α-form or β-form were examined, and the results are shown in Figure 2.

100ml of 63.5g/l sodium glutamate monohydrate (MSG) aqueous solution was prepared, and 1.7g sulfuric acid was taken up in a beaker. The quantity of glutamic acid was 0.034 mole and sulfuric acid was 0.017 mole. With varying the neutralizing volume in the first step, the MSG aqueous solution was kept at 25°C in a water bath while stirring by a magnetic stirrer, and the sulfuric acid was mixed in a moment. With varying time, the remaining MSG aqueous solution was added after the time to apply the supersaturation in the second step. At that time, overall rate of supersaturation was 4.0. The crystal forms were judged, and the results are shown in Figure 3.

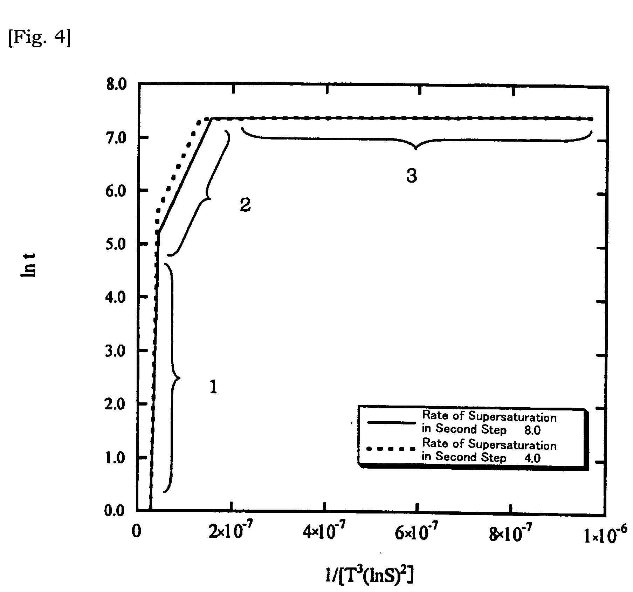

In order to confirm that the timing of the deposition of β-form crystals is generally said to be a crystallization waiting time by primary nuclei generation (the period from the formation of supersaturation to observation of deposition of crystals), the regions in Figure 2 and Figure 3 were arranged by the relationship between ln(t) (T: crystallization waiting time[sec.]) and $1/(T^3 \times (\ln S)^2)$(T: temperature [K], S: rate of supersaturation [-]), and the results are shown in Figure 4. As a result, it can be found that the boundary (1) and the boundary of (3) do not depend on the system. That is, it was found that each represents uniform primary nucleation or nonuniform primary nucleation, and β-form crystals are deposited as primary nuclei generation. The boundary (2) is considered to be transition region, and it was found that it can be expressed by almost a straight line, although it varies according to experimental conditions. This can be shown by the range described below.

In Figure 4, since it can be read that the boundary line (1) has a slope of $3.46 \times 10^8$ and ay intercept of -9.69, the line (1) can be described by the following formula;

[Mathematical 4]

$$\ln t = 1/(T^3(\ln S)^2) \times 3.46 \times 10^8 - 9.69$$

Since it can be read that the boundary line (2) has a slope of $1.95 \times 10^7$ and a y intercept of 4.35, the line (2)(full line) is expressed by:

[Mathematical 5]

$$\ln t = 1/(T^3(\ln S)^2) \times 1.95 \times 10^7 + 4.35$$

Since it can be read that the boundary line (2) (dotted line) has a slope of $1.95 \times 10^7$ and a y intercept of 4.80, the line can be expressed by:

[Mathematical 6]

$$\ln t = 1/(T^3(\ln S)^2) \times 1.95 \times 10^7 + 4.80$$

Since it can be read that the boundary line (3) has a slope of $6.16 \times 10^4$ and a y intercept of 7.34, it can be seen that the boundary line (3) is expressed by:

[Mathematical 7]

$$\ln t = 1/(T^3(\ln S)^2) \times 6.16 \times 10^4 + 7.34$$

By referring these to Figures 2 and 3, it can be seen that , in order to produce $\alpha$-form crystals stably, the remaining glutamic acid salt solution is added within:

in the case that the rate of supersaturation in the first supersaturated state is 2.6 to 8.0;

$$t \leqq \exp\{1/(T^3(\ln S)^2) \times 3.46 \times 10^8 - 9.69\}$$

in the case that the rate of supersaturation is 1.8 to 2.6;

$$t \leqq \exp\{1/T^3(\ln S)^2 \times 1.95 \times 10^7 + 4.35\}$$

in the case that the rate of supersaturation is 1.0 to 1.8;

$$t \leqq \exp\{1/T^3(\ln S)^2 \times 6.16 \times 10^4 + 7.34\}$$

While, in order to form the first supersaturated state, it is necessary to wait at least for 1 minute after the addition of acid to the glutamic acid salt solution, before adding the remaining glutamic acid salt solution.

The remaining glutamic acid salt solution may also be added at once, and at that time, it is preferable to be stirred.

It was found that the particle size distribution of crystals produced by this crystallization method is very fine compared with those produced by conventional method of adding seed crystals. In general, it is considered that, when produced crystals are used as seed crystals, the greater the surface area of the crystals is, the greater the effects are. This acts advantageously to supply seed crystals by this method in a big scale crystallization, and the like.

$\alpha$-form crystals slurry thus produced can be used as seed crystals in a big scale crystallization, and further, it is possible to conduct the following transition crystallization. That is, solubility of $\alpha$-form crystals is necessarily higher than solubility of $\beta$-form crystals in the range from 0°C to about 100°C. Hereupon, when seed crystals of $\beta$-form crystals are added or $\beta$-forum crystals occur suddenly at a certain probability, the solubility of the whole system becomes the solubility where liquid bottom body is $\beta$-form. Then, $\alpha$-form crystals begin to be dissolved, and all $\alpha$-form crystals are converted to $\beta$-form crystals utilizing the difference of solubility between $\alpha$-form crystal and $\beta$-form crystal as driving force. After $\beta$-form glutamic acid crystals are obtained, they are dissolved by adjusting to a pH forming monosodium glutamate monohydrate (MSG) by an aqueous sodium hydroxide solution, and concentrated to deposit crystals. The crystals are separated and dried to manufacture commercial products of sodium glutamate monohydrate (Non-Patent document 3).

[Example 1]

100ml of 127g/l monosodium glutamate monohydrate (MSG) aqueous solution was prepared, and 3.4g of sulfuric acid was taken up in a beaker. The quantity of glutamic acid was 0.068 mole and sulfuric acid was 0.034 mole. 46ml of the MSG aqueous solution was taken up, and kept at 25°C in a water bath while stirring by a magnetic stirrer, and the sulfuric acid was mixed in a moment to form a rate of supersaturation in the first step of 1.2. After 1.0 minute, the second step supersaturation was applied by adding the remaining MSG aqueous solution of 54 ml to complete crystallization. At that time, the overall rate of supersaturation was 8.0, and crystal form was $\alpha$-form.

[Comparative Example 1]

100ml of 127g/l monosodium glutamate monohydrate (MSG) aqueous solution was prepared, and 3.4g of sulfuric acid was taken up in a beaker. The quantity of glutamic acid was 0.068 mole and sulfuric acid was 0.034 mole. 54ml of the MSG aqueous solution was taken up, and kept at 25°C in a water bath while stirring by a magnetic stirrer, and the sulfuric acid was mixed in a moment to form a rate of supersaturation in the first step of 2.3. After 2.5minutes, the second step supersaturation was applied by adding the remaining MSG aqueous solution of 46 ml. At that time, the overall rate of supersaturation was 8.0, and both of $\alpha$-form crystals and $\beta$-form crystals deposited.

[Reference Example 1]

100ml of 127g/l monosodium glutamate monohydrate (MSG) aqueous solution was prepared, and 3.4g of sulfuric acid was taken up in a beaker. The quantity of glutamic acid was 0.068 mole and sulfuric acid was 0.034 mole. Sulfuric acid was gradually added to the MSG aqueous solution to adjust pH to 5.0, and hereupon, 1g of $\alpha$-form crystals were added as seed crystals. After stirring the solution for 30 minutes, the remaining sulfuric acid was added for 3 hours to deposit glutamic acid. The crystal form was $\alpha$-form.

Industrial Applicability

According to the invention, $\alpha$-form crystals of glutamic acid can be produced easily and stably, and can be incorporated into the manufacturing processes of glutamic acid.

**Claims**

1. A method of producing $\alpha$-form glutamic acid crystals which comprises the steps of:

   (A) forming a first supersaturated state of a glutamic acid salt solution having a rate of supersaturation of 1.0 to 1.7 by adding an acid at once to lower pH of the solution beyond isoelectric point of glutamic acid to pH 1.3 to 2.0 and waiting at least 1 minute after the addition of acid to the glutamic acid salt solution; and
   (B) forming a second supersaturated state of the solution by adding a glutamic acid salt solution within 25 minutes after the first supersaturated state is formed to shift the pH toward the isoelectric point.

2. The method of claim 1, which is performed without adding seed crystals.

3. The method of claim 1, which comprises

   preparing a glutamic acid salt solution and an acid with a volume capable of neutralizing the glutamic acid dissolved in the solution to its isoelectric point of pH 3.2,

dividing the glutamic acid salt solution into two portions,
using one of the portions as the glutamic acid salt solution in the step (A) to form the first supersaturated state by adding the above acid, and
using the other portion as the glutamic acid salt solution in the step (B).

4. A method of producing monosodium glutamate monohydrate which comprises transforming α-form glutamic acid crystals produced in the method of claim 1 to β-form glutamic acid crystals, dissolving the β-form glutamic acid crystals by sodium hydroxide aqueous solution to adjust to a pH forming monosodium glutamate monohydrate, concentrating to deposit crystals, and drying the deposited crystals.


**Patentansprüche**

1. Verfahren zum Herstellen von Glutaminsäurekristallen der α-Form, welches die folgenden Stufen umfasst:

   (A) das Erzeugen eines ersten übersättigten Zustandes einer Glutaminsäuresalzlösung mit einem Grad der Übersättigung von 1,0 bis 1,7, indem eine Säure auf einmal zugegeben wird, zur Verringerung des pH-Wertes der Lösung unter den isoelektrischen Punkt von Glutaminsäure auf einem pH-Wert von 1,3 bis 2,0 und mindestens einminütiges Warten nach dem Zugeben der Säure zu der Glutaminsäuresalzlösung; und
   (B) das Erzeugen eines zweiten übersättigten Zustandes der Lösung durch Zugeben einer Glutaminsäuresalzlösung innerhalb 25 Minuten nach der Bildung des ersten übersättigten Zustandes, um den pH-Wert zum isoelektrischen Punkt hin zu verschieben.

2. Verfahren nach Anspruch 1, das ohne das Zugeben von Keimkristallen durchgeführt wird.

3. Verfahren nach Anspruch 1, welches das Herstellen einer Glutaminsäuresalzlösung und einer Säure mit einem Volumen, das die in der Lösung aufgelöste Glutaminsäure zu ihrem isoelektrischen Punkt von pH 3,2 neutralisieren kann,
   das Teilen der Glutaminsäuresalzlösung in zwei Portionen, wobei eine der Portionen als die Glutaminsäuresalzlösung in der Stufe (A) zur Bildung des ersten übersättigten Zustandes durch Zugeben der vorstehend genannten Säure verwendet wird und die andere Portion als die Glutaminsäuresalzlösung in der Stufe (B) verwendet wird.

4. Verfahren zum Herstellen von Mononatriumglutamatmonohydrat, welches das Überführen der in dem Verfahren nach Anspruch 1 gebildeten Glutaminsäurekristalle der α-Form in Glutaminsäurekristalle der β-Form, das Auflösen der Glutaminsäurekristalle der β-Form durch eine wässrige Lösung von Natriumhydroxid zum Einstellen eines pH-Wertes, bei dem Mononatriumglutamatmonohydrat gebildet wird, das Konzentrieren der abgeschiedenen Kristalle und das Trocknen der abgeschiedenen Kristalle umfasst.


**Revendications**

1. Procédé de production de cristaux d'acide glutamique de forme α qui comprend les étapes suivantes :

   (A) formation d'un premier état sursaturé d'une solution de sel d'acide glutamique présentant un taux de sursaturation de 1,0 à 1,7 en ajoutant un acide en une seule fois pour abaisser le pH de la solution au-delà du point isoélectrique de l'acide glutamique au pH 1,3 à 2,0 et en attendant au moins 1 minute après l'addition de l'acide à la solution de sel d'acide glutamique ; et
   (B) formation d'un second état sursaturé de la solution en ajoutant une solution de sel d'acide glutamique dans les 25 minutes suivant la formation du premier état sursaturé pour déplacer le pH vers le point isoélectrique.

2. Procédé selon la revendication 1, qui est réalisé sans ajouter de germes cristallins.

3. Procédé selon la revendication 1, qui comprend
   la préparation d'une solution de sel d'acide glutamique et d'un acide ayant un volume capable de neutraliser l'acide glutamique dissous dans la solution à son point isoélectrique de pH 3,2,
   la division de la solution de sel d'acide glutamique en deux parties,
   l'utilisation de l'une des parties en tant que solution de sel d'acide glutamique à l'étape (A) pour former le premier état sursaturé en ajoutant l'acide indiqué ci-dessus, et

l'utilisation de l'autre partie en tant que solution de sel d'acide glutamique à l'étape (B).

4. Procédé de production de glutamate de monosodium monohydraté qui comprend la transformation de cristaux d'acide glutamique de forme α produits dans le procédé selon la revendication 1 en cristaux d'acide glutamique de forme β, la dissolution des cristaux d'acide glutamique de forme β par une solution aqueuse d'hydroxyde de sodium en vue de l'ajustement à un pH formant du glutamate de monosodium monohydraté, la concentration en vue de déposer des cristaux, et le séchage des cristaux déposés.

[Fig. 1]

[Fig. 2]

[Fig. 3]

[Fig. 4]

[Fig. 5]

(a) Glutamic acid crystals produced in Example 1 (α-form crystals alone)

(b) Glutamic acid crystals produced in Comparative Example 1 (mixture of $\alpha$ -form crystals and $\beta$ -form crystals; white fuzzy crystalline matters are $\beta$ -form crystals)

[Fig. 6]

(a) Glutamic acid crystals produced in Example 1 ($\alpha$-form crystals alone)

(b) Glutamic acid crystals produced in Comparative Example 1 (mixture of $\alpha$-form crystals and $\beta$-form crystals; white fuzzy crystalline matters are $\beta$-form crystals)

[Fig. 7]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 36017712 B
- JP 45004730 B
- JP 45011286 B
- CN 1312245 A
- GB 1118942 A

### Non-patent literature cited in the description

- **N. DOKI et al.** *Crystal Growth & Design,* 2004, vol. 4 (5), 949-953
- **JOCHEN SCHOLL et al.** *Chem. Eng. Technol.,* 2006, vol. 29 (2), 257-264
- Shokuhin Kogyo Handbook. Kenpokusha, 2004, 651-656
- Kagaku Binran. Maruzen, 1993, 432